**Europäisches Patentamt**

**European Patent Office**

**Office européen des brevets**

(11) Publication number : **0 514 544 B1**

# EUROPEAN PATENT SPECIFICATION

(45) Date of publication of patent specification :
01.06.94 Bulletin 94/22

(51) Int. Cl.[5] : **A61K 47/48, A61K 39/44, A61K 37/52**

(21) Application number : **91903666.5**

(22) Date of filing : **05.02.91**

(86) International application number :
**PCT/JP91/00136**

(87) International publication number :
**WO 91/12022 22.08.91 Gazette 91/19**

(54) **IMMUNE COMPLEXES.**

(30) Priority : 06.02.90 JP 27407/90
28.03.90 JP 80182/90
24.10.90 JP 287648/90

(43) Date of publication of application :
25.11.92 Bulletin 92/48

(45) Publication of the grant of the patent :
01.06.94 Bulletin 94/22

(84) Designated Contracting States :
AT BE CH DE DK ES FR GB GR IT LI LU NL SE

(56) References cited :
EP-A- 0 396 387
WO-A-85/00974
Chemical Abstracts, Volume 98, no. 21, issued
1983, May 23, (Columbus, 0hio, USA), T. Ter-
nynck et al.; page 484
Chemical Abstracts, Volume 100, no. 19, is-
sued 1984, May 7 (Columbus, 0hio, USA), D. A.
Vetterlein et al; page 218
Chemical Absracts, Volume 98, no. 17, issued
1983, April 25 (Columbus, 0hio, USA), D. Vetter-
lein et al.; page 249

(73) Proprietor : TAKEDA CHEMICAL INDUSTRIES,
LTD.
1-1, Doshomachi 4-chome
Chuo-ku, Osaka 541 (JP)

(72) Inventor : HAMAGUCHI, Naoru
2-6-704, Shirakawa 3-chome,
Ibaraki-shi
Osaka 567 (JP)
Inventor : SATO, Jun
2-1-514, Oogi 2-chome,
Higashinada-ku
Kobe-shi, Hyogo 658 (JP)
Inventor : DOKEN, Kazuhiro
11-10, Abikohigashi 3-chome
Sumiyoshi-ku, Osaka-shi, Osaka 5 (JP)
Inventor : IWASA, Susumu
21-2, Ohsumigaoka 1-chome,
Tanabe-cho,
Tsuzuki-gun
Kyoto 610-0 (JP)

(74) Representative : Laredo, Jack Joseph
Elkington & Fife
Prospect House
8 Pembroke Road
Sevenoaks, Kent TN13 1XR (GB)

## Description

The present invention relates to immune-complexes of biologically active substances which are therapeutically effective and monoclonal antibodies which can reduce the distribution volume in vivo of the biologically active substances about 0.5 times or less.

The distribution volume or volume of distribution is a measure of the apparent space in the body available to contain a drug or biologically active substance. The volume of distribution relates to the amount of drug in the body to the concentration of drug in the blood or plasma, depending upon the fluid measured [L. Goodman and E. Gilman, The Pharmacological Basis of Therapeutics, (1985), MacMillan Publishing Company, New York, NY pp. 22-28].

Biologically active substances are effective for treatment of human or animal diseases. However, if these substances are erroneously used, not only will the diseases not be treated, but serious side effects are produced. In some cases, such side effects cause death in humans or animals. Accordingly, when biologically active substances are given to treat human or animal diseases, it is necessary to take into careful consideration that the biologically active substances are present in appropriate concentrations at its sites of action only for suitable periods of time. For this purpose, the following methods are employed in many cases:

(1) Methods for using the biologically active substances are optimized,
(2) Medical prescriptions of the biologically active substances are improved, or
(3) The biologically active substances themselves are structurally modified to entirely different compounds.

Examples of the optimization of (1) include changes in administration courses (such as intravenous, subcutaneous, intramuscular and oral administrations). However, the distribution and half-life of the drugs can not be changed.

As examples of the improvements in medical prescriptions described in (2), liposomes and microcapsules can be used. In both of the cases, the drugs or drug solutions are encapsulated in vesicles and it is tried to keep suitable concentrations in vivo mainly by sustained release of the drugs. It is however generally difficult to change the half-life and distribution of the drugs themselves.

When the biologically active substances themselves are structurally modified as described in (3), the characteristics such as the half-life, the distribution and the metabolism in vivo can be changed. However, the pharmacological action of the biologically active substances themselves are occasionally entirely changed in quantity and quality.

For example, one such biologically active substance is interleukin-2 (hereinafter also briefly referred to as IL-2). IL-2 is a soluble protein formed by T cells activated by a lectin or an antigen, and enhances cellular immunity with the action of promoting proliferation and differentiation of NK cells, killer T cells, helper T cells and B cells. Hence, IL-2 is available for returning a depressed state of immunity to a normal state. This immunological activity of the IL-2 is useful for treatment of cancers, bacterial or viral infectious diseases, autoimmune diseases, immunodeficiency and the like.

However, IL-2 has a short biological half-life and a large distribution volume. For example, it is reported that the half-life of IL-2 in clearance from blood is 6.9 minutes when given to humans [M. Taotze et al., Journal of Immunology 135, 2865-2875 (1985)]. Although there are few examples in which the distribution volume of IL-2 is calculated, the initial concentration of the IL-2 in plasma is low when the IL-2 is given. While not wishing to be bound by theory, the low initial concentration of IL-2 in plasma may be explained by the resumption that the IL-2 is also distributed in extracellular fluid, as well as in plasma [C. Bindon et al., British Journal of Cancer 47, 123-133 (1983)]. Cytokines thus rapidly clear from organisms and are large in distribution volume. It is therefore anticipated that in clinical treatment situations IL-2 exhibits no satisfactory action or requires high doses. When the IL- 2 is given to organisms in large amounts, it is anticipated that the IL-2 is distributed throughout the overall organism, which results in increased side effects.

One way to improve such properties of the IL- 2 is to improve the therapeutic schedule. For example, it has been tried to increase the acting time in blood by increasing the number of administrations without changing the total dosage [Otsu et al., Cancer Immunology and Immunotherapy 30, 71-80 (1989)]. Further, for the purpose of increasing the half-life of the IL-2 in blood, the method of combining polyethlene glycol with the IL-2 has been proposed [Nishimura et al., Japanese Patent Unexamined Publication No. 60-226821/ 1985 which corresponds to EP-154,316 or N. Katre et al., PCT/US86/01252 (WO8700/056A)]. In these cases, however, the distribution volume is the same as that of the IL-2. It is therefore anticipated that the IL-2 is distributed throughout the overall organism, which results in no decrease in side effects, when the same amount of the IL-2 is given.

Urokinase, a biologically active substance, is prepared from human urine or tissue culture of human nephrocytes, and hydrolyzes plasminogen to form plasmin directly and dissolves fibrin. This substance is widely used in combination with anticancer drugs, or for treatment of cerebral thrombosis, peripheral arterial or venous obstruction and acute cardiac infraction. Urokinase is a direct activator to plasminogen, and usually used in a dose of about 2,400 to about 240,000 IU/day in Japan. With respect to humans, this is instantaneously or continuously injected into arteries or veins. High molecular weight urokinase, low molecular weight urokinase and prourokinase are known as urokinase.

In order to obtain the maximum drug effect, it is sometimes tried to improve therapeutic schedule to maintain urokinase blood concentrations constant without changing the total dosage. For example, as to humans, 10% of the total dosage is intravenously given instantaneously or for about 10 minutes, and the remaining 90% is further continuously injected into veins for 4 hours, whereby it is intended to maintain blood concentration constant to obtain the maximum effect. In this case, however, the distribution volume is the same as that of urokinase. It is therefore anticipated that urokinase is distributed throughout the overall organism, which results in no decrease in side effects, when the same amount of urokinase is given.

Considering that thrombus which are targets of urokinase exist in blood vessels, it is desirable to increase the half-life of urokinase, thereby significantly reducing the distribution volume about 0.5 time or less, to allow urokinase to exist in blood at high concentrations for a long period of time.

On the other hand, methods for producing such monoclonal antibody-interferon-$\alpha$ immune complexes as prolong the half-life of interferon-$\alpha$ by selecting monoclonal antibodies have been reported [Frinke et al., Japanese Patent Unexamined Publication No. 60-502104; PCT/US84/01389 (WO85/00974); and Cancer Research 45, 2421-2424 (1985)]. Japanese Patent Unexamined Publication No. 60-502104 and WO85/00974 described above show that the distribution volume is 20.8 ml when interferon-$\alpha$ is intravenously given, and that this distribution volume is little different from the distribution volume of 19.2 ml when the monoclonal antibody-interferon-$\alpha$ immune complex is given. Cancer Research described above reports that the distribution volume is 11.9 ml when the monoclonal antibody-interferon-$\alpha$ immune complex is intravenously given, compared to that the distribution volume is 17.2 ml when interferon-$\alpha$ is intravenously given. In case of the immune complex, therefore, the distribution volume is only reduced 0.69 times. This degree of reduction in distribution volume results in an insufficient increase in drug effect.

Pharmacokinetics is often used as a method for analyzing the relationship between adsorption, distribution, and elimination of biologically active substances in vivo. The resulting parameters represent the relations between the distribution, concentrations and times of the biologically active substances in vivo. For example, when a biologically active substance is intravenously given and the blood concentration of the biologically active substance is pharmacokinetically analyzed by the most idealized one compartment model, in which all drug administration occurs directly into a single compartment, it is possible to calculate the distribution volume relating to the distribution in vivo of the biologically active substance and the elimination rate constant relating to the half-life.

The bispecific antibody, a kind of monoclonal antibody, has already been tried in the method of Frinke et al. described above and in other methods. However, no antibody which changes the distribution volume in vivo has been reported yet.

If a biologically active substance-monoclonal antibody immune complex can be obtained which can decrease the distribution volume of the biologically active substance more significantly than the biologically active substance alone, the blood concentration can be increased to exhibit the effect thereof more effectively and the side effects can be reduced.

SUMMARY OF THE INVENTION

The present inventors have studied various monoclonal antibody-biologically active substance complexes which can reduce the distribution volume of biological active substances when given to organisms, more than when the substances are given alone. As a result, the present inventors have obtained the information that immune complexes which can reduce the distribution volume of biologically active substances 0.5 times or less can increase the blood concentration in vivo of the biologically active substances to increase the drug effect significantly, and have succeeded in obtaining such immune complexes, thus completing the present invention.

The present invention provides an immune complex comprising a biologically active substance and a monoclonal antibody, the monoclonal antibody being able to reduce the distribution volume of said substance 0.5 times or less compared to the case in which said substance is used alone.

BRIEF DESCRIPTION OF THE DRAWINGS

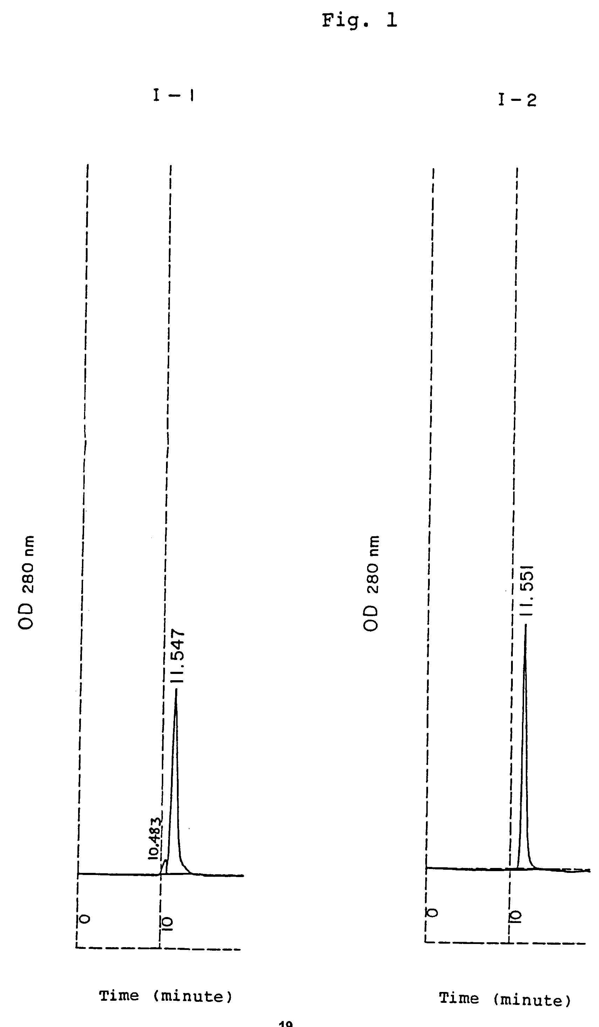

Figs 1-1 and 1-2 are graphs showing chromatograms obtained by high performance liquid chromatography (HPLC) in Example 2. Namely, Fig. 1-1 is a graph showing a hromatogram obtained when commercial mouse IgG is applied to HPLC using a GS-520 column. Fig. 1-2 is a graph showing a chromatogram obtained when a purified anti-rhIL-2 monoclonal antibody is applied to HPLC using a GS-520 column;

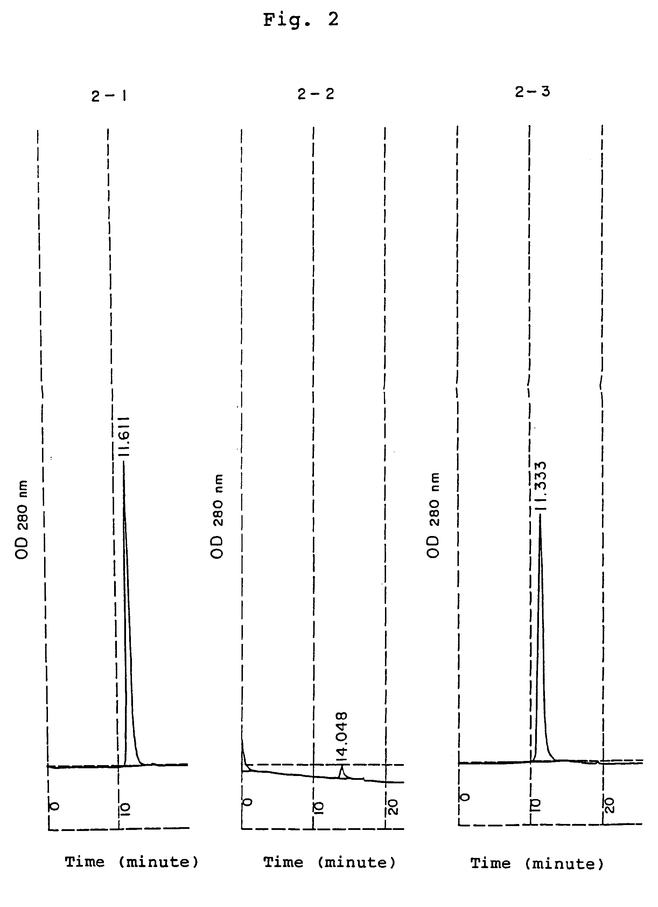

Figs. 2-1, 2-2 and 2-3 are graphs showing chromatograms obtained by HPLC using a GS-520 column in Example 3. Namely, Figs. 2-1, 2-2 and 2-3 show the results of the anti-rhIL-2 monoclonal antibody; rhIL-2 and an immune complex of the rhIL-2 and the anti-rhIL-2 monoclonal antibody (mole ratio: 1:2), respectively;

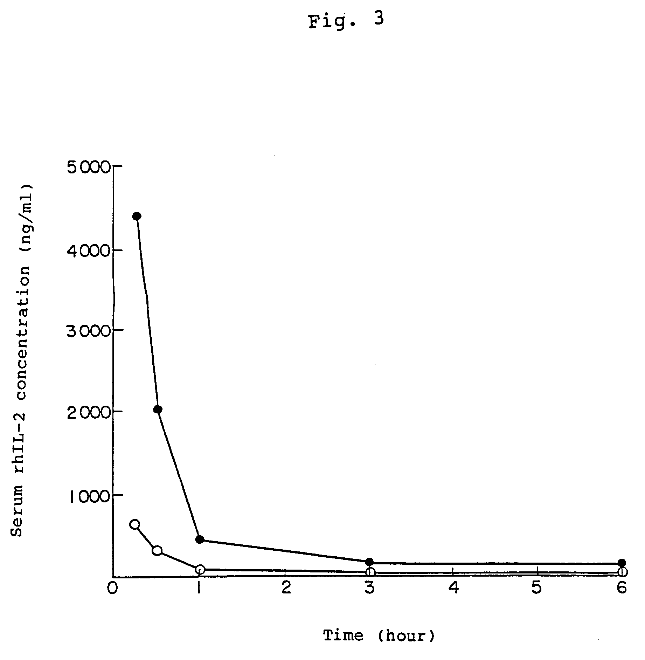

Fig. 3 is a graph showing the relationship between the serum rhIL-2 concentration and the time obtained when a mixed solution (weight ratio: 1:5) of the rhIL-2 and unspecific mouse IgG is given and when the rhIL-2-monoclonal antibody immune complex is given, respectively, in Experimental Example 1;

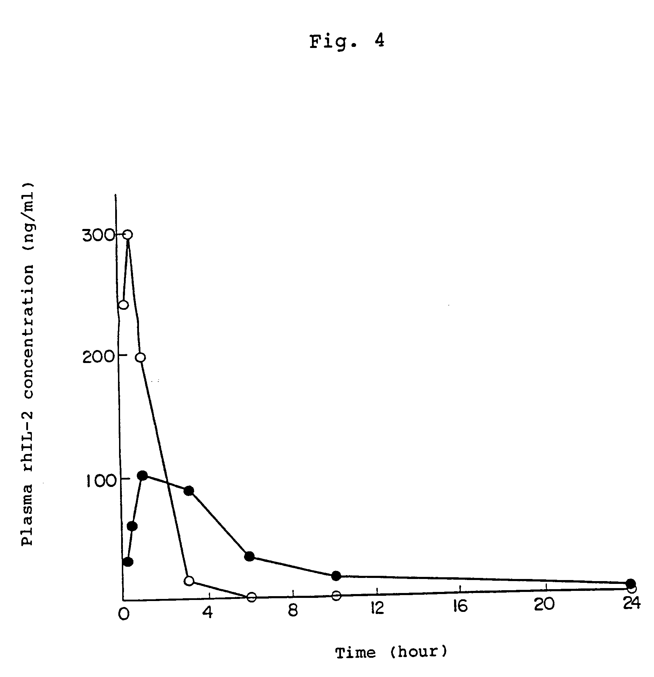

Fig. 4 is a graph showing the relationship between the plasma rhIL-2 concentration and the time obtained when a mixed solution (weight ratio: 1:5) of the rhIL-2 and the unspecific mouse IgG is given and when the rhIL-2-monoclonal antibody immune complex is given, respectively, in Experimental Example 3;

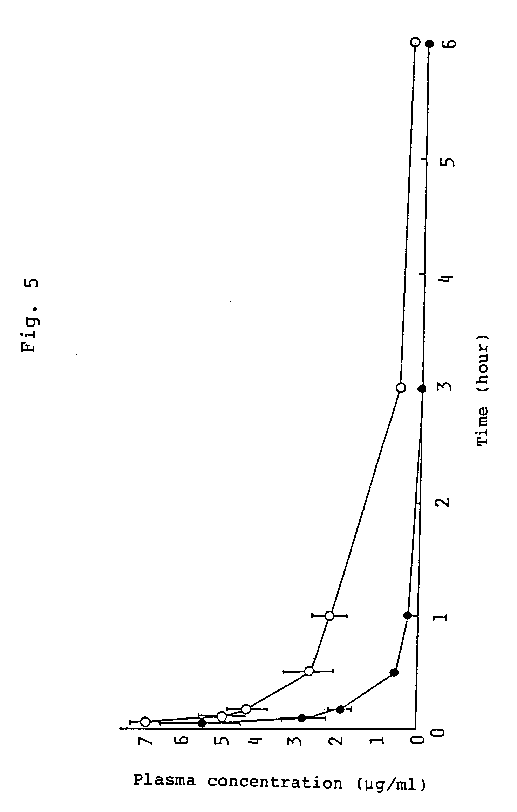

Fig. 5 is a graph showing the relationship between the plasma urokinase concentration and the time obtained when an urokinase-monoclonal antibody immune complex is given and when urokinase is given alone, respectively, in Experimental Example 6; and

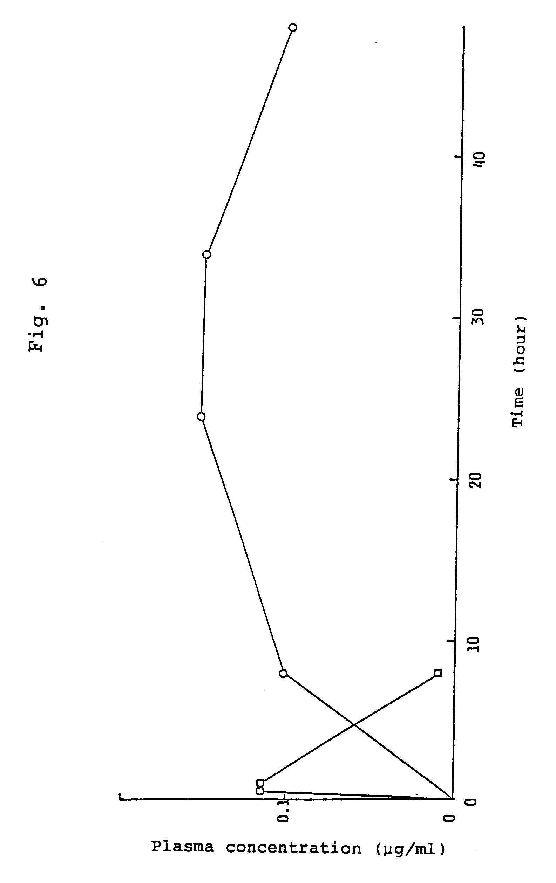

Fig. 6 is a graph showing the relationship between the plasma urokinase concentration and the time obtained when the urokinase-monoclonal antibody immune complex is given and when urokinase is given alone, respectively, in Experimental Example 7.

## DESCRIPTION OF THE PREFERRED EMBODIMENTS

The biologically active substances are substances having biological activity which are necessary to maintain life activity of organisms, to keep homeostasis and to develop the activity of organisms. In the case of disease, it is possible to improve the functions of the organisms to normal conditions by the use of such biologically active substances.

The biologically active substances used in the present invention include, for example, proteins such as cytokines, cell growth factors, hormones and enzymes, peptides, and mutants and fragments thereof having biological activity. The substances also include antagonists and agonists thereof. They further include steroids, antagonists and agonists thereof, and arachidonate cascades.

Examples of cytokines include interleukin-1 obtained by activating a macrophage with an antigen, interleukin-2(IL-2) obtained by activated T cells with an antigen, interleukin-3 produced by a specific clone of T cells, interleukin-4 produced by T cells, T cell replacing factor (TRF), B cell differentiating factor (BCDF), antigenic specific suppressor factor (TsF), soluble immune response inhibiting factor (SIRF), suppressor inducing factor (SIF), interferon-$\gamma$ (IFN-$\gamma$), B cell growth factor (BCGF) produced from B cells, B cell differentiation augmenting factor (BCDF), B cell growth inhibiting factor (SBF), macrophage activating factor (MAF) which is said to be produced from B cells or T cells, macrophage migration inhibition factor (MIF), leukocyte migration inhibition factor (LIF), lymphotoxin (LT), interferon-$\alpha$ (INF-$\alpha$), granulocyte colony stimulating factor (G-CSF), macrophage colony stimulating factor (GM-CSF), monocyte colony stimulating factor (M-CSF) and interferon-$\beta$ (IFN-$\beta$) produced from fibroblasts. In addition, cytokines also include interleukins such as interleukin-5 and interleukin-6, chemotactic factors such as macrophage chemotactic factor (MCF) and lymphocyte chemotactic factor (LCF), inflammatory lymphokines such as vascular permeability factor (VPF), perforin produced from cytotoxic T cells and tumoricidal factors such as lymphocyte-derived lymphotoxin.

The cell growth factors mainly include platelet-derived cell growth factor (PDGF) mainly targeting fibroblasts and smooth muscle cells, epithelial cell growth factor (EGF) mainly targeting fibroblasts, smooth muscle cells, vascular endotherial cells, epithelial cells and chondrocytes, fibroblast growth factor (FGF) mainly targeting fibroblasts, smooth muscle cells, vascular endotherial cells and epithelial cells, nerve growth factor (NGF) mainly targeting nerve cells, nerve growth factor (IGF-I and IGF-II) mainly targeting chondrocytes, and erythropoietin proliferating erythrocytes.

Examples of the hormones include tissue plasminogen activators (TPA), insulin, angiotensin I, angiotensin II, angiotensin III, angiotensin II inhibitors, atrial sodium diuretic peptides, bradykinin, calcitonin, corticotropin, dynorphin, kyotorphin, endorphin, enkephalin, secretin, growth hormone-releasing factor (GRF), luteinizing hormone-releasing hormone (LH-RH), neurotensin, parathyroid hormone (PTH), oxytocin, vasopressin, vasotocin, somatostatin, thyrotropin- releasing hormone (TRH), human growth hormone, thyroid- stimulating hormone and prolactin. Examples of the agonists thereof include leuprorelin.

Examples of the steroids include progesterone, estrogen, adrenocortical hormone, aldosterone, dexamethasone, estrone, estradiol and dihydrotestosterone. Examples of the antagonists of the steroids include oxenedorone which is an antagonist of dihydrotestosterone.

Examples of the arachidonate cascades include prostaglandin $E_2$.

The enzymes include urokinase, superoxide dismutase, asparaginase and adenosine deaminase.

As the biologically active substances, cytokines and enzymes are preferable. In particular, IL-2, urokinase and prourokinase are preferably used. The IL-2 will hereinafter be described in detail.

When the IL-2 is used as the biologically active substance, any may be used as long as it is derived from a mammal. The entire molecule of the IL-2 or partial fragment peptides may also be used. The IL-2 defined here includes human cell-derived IL-2 complexes and IL-2 complexes obtained from microorganisms or animal cells by genetic engineering techniques, and is not limited by manufacturing methods. The IL-2 also includes substances having original human IL-2 complexes as basic molecular skeletons such as substances in which the amino acids are partly substituted by genetic engineering techniques and substances in which amino acid residues are added to or deleted from the N-terminal sides and/or the C-terminal sides.

More particularly, the IL-2 includes substances having activity similar to that of the IL-2, namely substances having the activity of enabling the passage maintenance of T cells while maintaining the functions thereof. Specifically, there may be used, for example, polypeptide (I) (human IL-2) having the amino acid sequence shown in Fig. 1 of Japanese Patent Unexamined Publication No. 61-78799/1986 which corresponds to EP-176,299 and a polypeptide having a portion of the amino acid sequence necessary for its biological or immunological activity. Examples of such polypeptides include a polypeptide lacking one amino acid residue at the amino terminus of the polypeptide (I) (European Patent Publication No. 91539), a polypeptide lacking 4 amino acid residues at the amino terminus thereof (Japanese Patent Unexamined Publication No. 60-126088/1985) and a polypeptide lacking several amino acid residues at the carboxyl terminal portion. Further, a portion of the constituent amino acid residues of the above polypeptide (I) may be deleted or substituted by a different amino acid or different amino acids. For example, the cystine residue at the 125-position may be replaced with a serine residue (Japanese Patent Unexamined Publication No. 59-93093/1984 which corresponds to U.S. Patent No. 4,518,584).

In particular, human IL-2 having the amino acid sequence shown in Fig. 1 of Japanese Patent Unexamined Publication No. 61-78799/1986 which corresponds to EP-176,299 is preferably used. In this case, the IL-2 may be a mixture of a polypeptide having a methionine residue (Met) at the amino terminus and a polypeptide having no Met (Japanese Patent Unexamined Publication No. 60-115528/1985 which corresponds to EP-145,390 and Japanese Patent Unexamined Publication No. 61-78799/1986) or a polypeptide having no Met at the amino terminus and starting from an alanine residue (Ala) (Japanese Patent Unexamined Publication No. 61-78799/1986). Further, the IL-2 may be a polypeptide having a sugar chain or sugar chains.

Next, urokinase will hereinafter be described. High molecular weight urokinase, low molecular weight urokinase and prourokinase are known as urokinase. Other urokinase fragments and urokinase derived from mammals other than humans may also be used. The urokinase defined here is not limited to urokinase obtained from microorganisms or animal cells by genetic engineering techniques. The urokinase also includes substances having original urokinase as basic molecular skeletons such as muteins in which the amino acids are partly substituted by genetic engineering techniques and substances in which amino acid residues are added to or deleted from the N-terminal sides and/or the C-terminal sides.

The monoclonal antibodies used in the present invention are used as the immune complexes with the biologically active substances. Any monoclonal antibody may be used as long as it can reduce the distribution volume in vivo of that substance about 0.5 times or less, when the immune complex is given to organisms.

The monoclonal antibodies used in the present invention may extend or not extend the blood half-life when they are used as the immune complexes.

The immune complexes of the present invention are expected to reduce the side effects of the biologically active substances, because the distribution volume is decreased 0.5 time or less.

Furthermore, in the immune complexes of the present invention, the immune complexes extending the half-life are expected to continue the effect of the biologically active substances.

The monoclonal antibodies are obtained by producing hybridoma cells for producing the monoclonal antibodies which are obtained by using the biologically active substances or derivatives thereof as immunogens, and then cultivating the hybridoma cells.

In preparing the monoclonal antibody producing hybridoma cells used in the present invention, any hybridoma cells may be used as long as they produce monoclonal antibodies which are specific for the biologically active substances, form the immune complexes with the biologically active substances and can reduce the distribution volume of the biological active substances when the complexes are given to organisms, more than when the substances are given alone.

The monoclonal antibodies may neutralize the biological activity of the biological active substances.

The immunogens used for preparing the hybridoma cells include the above-mentioned biologically active substances and derivatives thereof (for example, mutants and fragments thereof acting similarly to the above substances); and antagonists and agonists of the above substances and derivatives thereof (for example, mutants and fragments thereof acting similarly thereto). In the derivatives, the fragments are particularly preferable.

As the immunogens, heat-denatured biologically active substances may be used and are preferably used in some cases.

Animals (for example, rabbits, rats, mice and guinea pigs) are immunized with the above-mentioned immunogens as they are or combined with carrier proteins to obtain antibody producing cells.

Examples of such carrier proteins include bovine serum albumin (BSA), ovalbumin (OVA), keyhole limpet hemocyanin (KLH) and thyroglobulin.

For combination of the biologically active substances with the carrier proteins, for example, cysteine portions of the biologically active substances are used. Namely, the carrier proteins are previously, for example, maleimidated by N-($\gamma$-maleimidobutyryloxysuccinimide (hereinafter also briefly referred to as GMBS) or dithiopyridylated by N-succinimidyl-3-(2-pyridyldithio)propionate (hereinafter also briefly referred to as SPDP), whereby the above biologically active substances can be chemically combined with the carrier proteins through the SH groups of Cys residues contained in the biologically active substances.

The antibody producing hybridoma cells are prepared by the method of immunizing animals with the above biologically active substances, the derivatives thereof or the condensed products thereof with the carrier proteins, according to known methods and fusing the resulting antibody producing cells with myeloma cells. As the immunizing animals, for examples, rabbits, rats, mice and guinea pigs are used. When the monoclonal antibodies are produced, however, mice are particularly preferable. Inoculation is performed according to known methods. For example, about 1 to 100 $\mu$g, preferably about 10 to 25 $\mu$g emulsified in the same volume (0.1 ml) of saline and Freund's complete adjuvant is inoculated into each of the mice subcutaneously at its back portion or intraperitoneally once every 2 to 3 weeks, about 3 to 6 times in all.

Individual animals having a high antibody titer are selected from these immunizing animals such as mice, and the spleens and/or the lymphatic cells are collected therefrom about 3 to 5 days after the final immunization. Then, the antibody producing cells contained therein are fused with the myeloma cells. The fusing procedure may be conducted according to known methods. Fusion accelerators include polyethylene glycol (hereinafter also briefly referred to as PEG) and Sendai virus. In particular, PEG is preferably used. The myeloma cells include NS-1, P3U1 and SP2/0, and particularly, P3U1 is preferably used. For example, the ratio of the number of the spleen cells to that of the myeloma cells is preferably about 1:1 to 10:1. It is preferred that PEG having a molecular weight of about 1,000 to 6,000 is added thereto at a concentration of about 10 to 80% and incubation is carried out at about 20 to 37°C, preferably at about 30 to 37°C for about 3 to 10 minutes.

In the present invention, the hybridoma cells can be screened by various methods known in the art. For example, the antibody titer of a culture supernatant is measured by an enzyme immunoassay (hereinafter also briefly referred to as EIA) which comprises allowing the biologically active substance to be adsorbed by a microtiter plate, and then, adding the hybridoma culture supernatant thereto to detect an anti-biologically active substance specific antibody combined with the plate. The hybridoma cells having positive antibody activity which have been selected and bred in HAT (hypoxanthine, aminopterin and thymidine) medium are immediately subjected to cloning. Usually, this is easily carried out by a limiting dilution analysis. The cloned hybridoma cells are selected, whereby the desired monoclonal anti-biologically active substance specific antibody producing hybridoma cells can be obtained.

Pharmacokinetic parameters given when the biologically active substance or a combination of the biologically active substance and the monoclonal antibody are administered can be determined by methods known in the art. Namely, an aqueous solution of the biologically active substance is intravenously given to animals, and the blood is collected after a specified period of time from administration. After centrifugation, the concentration of the biologically active substance in the resulting plasma is determined, and this result is analyzed by using pharmacokinetic parameter analyzing software such as Automated Pharmacokinetic Analysis System(APAS), whereby the distribution volume Vd can be obtained.

For example, 0.1 ml of a saline solution of the immune complex containing 10 $\mu$g of recombinant IL-2 (rIL-2) is intravenously given to mice, and 15 and 30 minutes and 1, 3 and 6 hours after dosing, the blood is collected from their supraorbital veriplex with a heparinized capillary tube. After centrifugation, the concentration of the rIL-2 in the resulting plasma is assayed by EIA. The plasma rIL-2 concentration can be approximated by the following one compartment model formula, using pharmacokinetic parameter analyzing software for the plasma concentration such as APAS [K. Yamaoka and Y. Tanigawara, Primer of Pharmacokinetics by Microcomputers, p.159, Nankodo (1983)]:

$$C = C_\theta e^{-kt}$$

wherein C represents the plasma concentration, $C_\theta$ represents the initial plasma concentration obtained by the extrapolation of the time to 0, t represents the time after dosing, and k represents the elimination rate constant. Further, the distribution volume Vd can be obtained by dividing the dose by $C_\theta$. The half-life can be obtained by dividing 0.693 by k.

To obtain the distribution volume Vd, the measurements are plotted on semilog graph paper with plasma concentration as ordinate and time as abscissa, and the dose is divided by the intercept (plasma concentration) of the ordinate when the plotted line is approximated by a straight line. The half-life can be obtained by determining the time it takes for the plasma concentration to be reduced by 50%.

Using rats in lieu of mice as the animals to be administered, it is also possible to give the immune complex containing 100 μg of the rIL-2 similarly. The blood may be withdrawn, for example, from caudal tail veins, as long as usual methods are employed. Also when serum is used in lieu of plasma, the parameters can also be determined.

As to the biologically active substances other than the rIL-2 which are defined in this invention, the parameters can be determined similarly to the above method.

In the present invention, the monoclonal antibodies are used which can reduce the distribution volume in vivo of the biologically active substances about 0.5 times or less, compared to the case in which the substances are used alone. Further, the antibodies are used which can reduce the distribution volume preferably about 0.05 to 0.5 times, more preferably about 0.1 to 0.5 times, and particularly preferably about 0.1 to 0.3 times.

The distribution volume and the clearance half-time are obtained by thus analyzing the concentration of the biologically active substances in blood, serum or plasma with time when the substancs are intravenously given to the animals, and they reflect the distribution, the elimination, the metabolism and the like of the biologically active substances. Thus, the pharmacokinetic parameters are determined by the relationship between the organisms and the biologically active drugs, and have nothing to do with the route of administration. In other words, the pharmacokinetic parameters generally show the characteristics in the distribution, the elimination, the metabolism and the like of the biologically active substances, even if routes of administration other than intravenous administration, for example, subcutaneous injection, intramuscular and oral routes of administration, are employed.

The above-mentioned monoclonal antibody producing hybridoma cells are usually cultivated in a liquid medium or in a peritoneal cavity of an animal (for example, a mammal such as a mouse) according to methods known in the art. The antibody contained in the culture solution or in the ascites can be purified by using biochemical techniques in combination. For example, the cell culture solution or the ascites is centrifuged to take out a supernatant, which is subjected to salt precipitation (using ammonium sulfate or sodium sulfate usually). The resulting protein precipitate is dissolved in an appropriate solvent, and the resulting solution is dialyzed. Then, the desired antibody can be isolated and purified by column chromatography such as ion exchange chromatography, gel permeation chromatography, protein A chromatography or hydroxyapatite column chromatography. It is also possible to obtain the antibody by filtering the supernatant through a 5-μm filter in lieu of salt precipitation, replacing the solvent with an appropriate solvent by gel filtration, and then, subjecting to column chromatography similar to that described above. By the isolating and purifying procedures as described above, for example, about 3 to 20 mg and further about 5 to 200 mg of the monoclonal antibody having a purity of at least 80% in weight ratio of protein and further a purity of at least 90% can be obtained from 1 liter of the culture supernatant. And about 5 to 300 mg of similar antibody may be obtained from 20 ml of ascites and sometimes 3 to 100 mg or about 3 to 20 mg of antibody may be obtained.

The monoclonal antibody obtained as described above is homogeneous as a protein.

It is preferred that the above monoclonal antibody is immunoglobulin G. Further, the monoclonal antibodies used in the present invention may be fragments such as Fab, Fab′ and F(ab′)$_2$ produced by treating IgG with proteases such as pepsin. The fragments have the ability to bind to the biologically active substances.

The monoclonal antibodies constituting the immune complexes of the present invention are selected from immune complexes obtained by mixing the various monoclonal antibodies prepared according to the above-mentioned manufacturing methods with the biologically active substances, for example, in phosphate buffered saline to give a mole ratio of about 1:2, and hybridoma cells which produce the monoclonal antibodies are further selected. The distribution volume at this time can be determined by the methods described above.

In selecting the monoclonal antibodies, it is preferred to select the antibodies so that the ratio of the immune complexes comprising the monoclonal antibodies and the biologically active substances to that of the biologically active substances become 1:2 or less.

When this monoclonal antibody is mouse IgG, a DNA sequence coding for a variable region containing an antigen recognition site of the antibody is obtained, and a gene coding for a constant region of human IgG is combined therewith by using the gene manipulation technique [Z. Steplewski et al., Proc. Natl. Acad. Sci. USA 85, 4852 (1988)], whereby a mouse-human chimera antibody can be prepared. Such a chimera antibody is advantageously given to humans because of its low antigenicity.

The required immune complexes of the present invention can be obtained by mixing the anti-biologically active substance monoclonal antibodies with the biologically active substance. The mole ratio of the antibodies to the biologically active substances to obtain the immune complexes is preferably about 1:2. When the complexes are given, however, it is also possible to vary the mole ratio from about 10:1 to about 1:50, preferably from about 5:1 to about 1:50, by increasing the amount of the anti-biologically active substance monoclonal antibodies or the biologically active substances. The mole ratio is preferably about 10:1 to about 1:10, more preferably about 1:1 to about 1:10, and most preferably about 5:1 to about 1:5. As mixing methods, it is preferred that they are mixed in aqueous solution form to produce the immune complexes. These methods substantially include the method of dissolving a mixture of the powdery anti-biologically active substance monoclonal antibody and the biologically active substance which is previously prepared, before dosing, to form the immune complex. The dissolved immune complexes may be solidified by vacuum drying, and dissolved when used. The mixing methods further include the method of giving the anti-biologically active substance monoclonal antibody and the biologically active substance separately to organisms, and substantially forming the immune complex in the organisms.

Furthermore, the present invention provides biologically active drugs or pharmaceutical compositions containing the above-mentioned immune complexes.

Namely, the immune complexes of the present invention obtained as described above can be used as liquid injections as such, or after mixing them with pharmaceutically acceptable excipients, diluents and the like, followed by mechanical sterilization through membrane filters, if necessary. Also, solutions of the immune complexes, after mixing them with pharmaceutically acceptable excipients, diluents and the like, followed by mechanical sterilization through membrane filters, if necessary, are vacuum dried to prepare powder for injections, which are dissolved in water or saline when used.

Further, solutions containing only the anti-biologically active substance monoclonal antibodies can be subjected to procedures similar to those described above to prepare liquid injections and powder for injections.

As the excipient, for example, mannitol can be used. As the diluent, water is usually employed. Characteristics as injections can be improved by appropriately incorporating the above excipients, stabilizers, soothing agents, preservatives and the like.

Examples of the above stabilizers include human serum albumin (HSA). Isotonic agents include, for example, sodium chloride, glucose, mannitol and sorbitol. Examples of pH regulating agents include hydrochloric acid and sodium hydroxide. Examples of the stabilizers include sodium pyrosulfite, Rongalit and sodium hydrogenmetasulfite. Examples of the soothing agents include xylocaine hydrochloride, chlorobutanol and procaine hydrochloride. The preservatives include, for example, benzyl alcohol, phenol, methyl paraoxybenzoate and propyl paraoxybenzoate.

Although these immune complexes can be used as solution preparation, the complexes powdered by vacuum drying can also be dissolved in using them. Powdered preparations are preferable because of their easy handling. Examples of the vacuum drying include lyophilization. When lyophilization is carried out, it is preferred that samples cooled to -10°C or below are lyophilized in flasks in laboratories, and in trays or vials industrially.

The samples can also be dried by using a SpeedVac Concentrator (SAVANT) in lieu of lyophilization. In this case, the temperature is preferably about 0 to 30°C. The vacuum pressure is about 20 mmHg or less, and preferably about 10 mm Hg or less.

It is preferred to adjust the pH to about 3.0 to 10.0 by adding salts having buffer action such as phosphates to the solutions to be dried. In order to make the osmotic pressure of injections at the dosing time isotonic with organisms, it is preferred that the isotonic agents usually used for injections, such as sodium chloride and glucose, are previously added to the solutions. However, it is possible to dissolve the samples in salt solutions, glucose solutions or plasma substitutes having an appropriate osmotic pressure when they are used, without adding the isotonic agents to the solutions before drying. Stabilizers such as thimerosal may also be added if necessary. For the purpose of stabilizing proteins, it is also possible to add human serum albumin (HSA) as the stabilizer.

The dried preparations thus obtained are stable for a long period of time, and can be dissolved in water, salt solutions, glucose solutions or plasma substitutes to prepare injections when used.

Further, the above-mentioned carriers, stabilizers and the like may be added to injections.

Thus, it is preferred that the preparations of this invention are dried powdery products.

According to the methods described above, the immune complexes of the therapeutically effective biologically active substances and the monoclonal antibodies which can reduce the distribution volume of the above substances are obtained.

Also, the biologically active drugs containing the above immune substances are obtained.

The monoclonal antibodies used for the immune complexes of the present invention are significantly low in toxicity, so that the immune complexes are also low in toxicity.

The immune complexes of the present invention can be used for purposes similar to those of the biologically active substances constituting the immune complexes. For example, when the biologically active substances can be used for treatment of cancer, immunodeficiency, inflammation, hormone control abnormality, hypertension, cardiac disease or the like, the immune complexes of the present invention can be used for a purpose similar to that described above.

Accordingly, the immune complexes of the present invention or the drugs containing them can be given to mammals such as mice, rats, dogs, cats, pigs, cattle, monkeys and humans intravenously, subcutaneously, intramuscularly or intraperitoneally.

The immune complexes of the present invention are given in such a dosage that the dosage of the biologically active substances amounts to a known dosage or less. The dose may be adjusted as appropriate and, ultimatly, will be determined by the attending physician or veterinarian. Such a dose of the claimed immune complexes is referred to herein as an "effective amount".

When the anti-biologically active substance monoclonal antibody and the biologically active substance are separately given to organisms to substantially form the immune complex in the organisms, the above monoclonal antibody which has been formed into an injection as described above is previously given to mammals such as mice, rats, dogs, cats, pigs, cattle, monkeys and humans intravenously, subcutaneously, intramuscularly or intraperitoneally, and then, the biologically active substance which has been formed into an injection as described above is given intravenously, subcutaneously, intramuscularly or intraperitoneally. In this case, the two routes of administration may be different from each other.

The mole ratio of the anti-biologically active substance monoclonal antibodies to the biologically active substances is preferably about 10:1 to about 1:50, more preferably about 5:1 to about 1:50, and most preferably about 1:1 to about 1:10 or about 5:1 to about 1:5. Although the dosage of the biologically active substances or the anti-biologically active substance monoclonal antibodies varies depending on the subject disease, symptom, route of administration and the like, the biologically active substances are given in a known dosage or less.

For example, when the biologically active substance has anti-cancer action and the immune complex of the present invention is intravenously given to cancer patients, for humans the biologically active substance is used in an amount of about 0.1 µg to 10 mg per human, and preferably in an amount of 1 µg to 1 mg per human, for other mammals the substance is used in an amount of about 0.002 µg to 0.2 mg per kg, and preferably in an amount of 0.02 µg to 0.02 mg per kg.

When the biologically active substance is urokinase and the complex is intravenously given to thrombosis patients, the urokinase is used in an amount of about 0.01 to 0.5 mg/kg daily, and preferably in an amount of about 0.02 to 0.2 mg/kg daily.

The immune complexes of the present invention reduced 0.5 times or less in vivo distribution volume of the biologically active substances, so that the blood concentration is increased to enhance the effectiveness of the biologically active substances significantly. In addition, the side effects are reduced by leaving the biologically active substances in the vessel system, mainly in blood.

Various diseases such as cancer, immunodeficiency, inflammation, hormone control abnormality, hypertension and cardiac disease can be more effectively treated by giving the immune complexes to mammals.

In cases of urokinase and prourokinase, the urokinase monoclonal antibody immune complexes can be obtained which prolong the elimination half-life of the drugs in vivo without impairing their biological activity and significantly reduce the distribution volume 0.5 times or less, compared to the case in which urokinase or prourokinase is used alone. According to the present invention, the blood concentration can be kept higher, and the effect thereof can be exhibited more effectively. Further the side effects can be reduced.

The present invention will hereinafter be described in detail with the following Examples, Reference Examples and Experimental Examples.

rhIL-2 used in the following Examples and Experimental Examples is a mixture of one having a methionine residue at the N-terminus and one having no methionine residue, which are produced by a method similar to that described in Japanese Patent Unexamined Publication No. 60-115528/1985 which corresponds to EP-145390 or Japanese Patent Unexamined Publication 61-78799/1986 which corresponds to EP-176299, using transformant Escherichia coli N4830/pTB285 (IFO 14437, FERM BP-852).

Transformant E. coli N4830/pTB285 described above was deposited with the Institute for Fermentation, Osaka, Japan (IFO) under the accession number IFO 14437 on April 25, 1985. This microorganism was also deposited with the Fermentation Research Institute, Agency of Industrial Science and Technology, Ministry of International Trade and Industry, Japan (FRI) under the accession number FERM P-8199 on April 30, 1985. This deposit was converted to the deposit under the Budapest Treaty and the microorganism has been stored at FRI under the accession number FERM BP-852.

Mouse hybridoma cell HRL1-52 obtained in Example 1 mentioned below was deposited with the IFO under the accession number IFO 50222 on January 29, 1990. This hybridoma cell was also deposited with the FRI under the accession number FERM BP-2747 on January 31, 1990.

Hybridoma cells used in Reference Examples mentioned below can be produced by the method described in European Patent Publication No. 363712, and were deposited as follows.

| Animal Cell | IFO<br>IFO No. | FRI<br>FERM No. |
|---|---|---|
| Mouse hybridoma UK1-3 | 50176<br>(September 21, 1988) | BP-2083<br>(October 4, 1988) |
| Mouse hybridoma UK1-87 | 50177<br>(September 21, 1988) | BP-2084<br>(October 4, 1988) |
| Mouse hybridoma UK1-6 | 50208<br>(August 9, 1989) | BP-2548<br>(August 11, 1989) |

Example 1 Preparation of Mouse Anti-Human IL-2 Monoclonal Antibody Producing Hybridoma Cell

(1) Preparation of Immunogen

A 1 mg/ml saline solution of rhIL-2 was heat treated at 90°C for 3 minutes to prepare denatured human IL-2. After storing in the frozen state, it was used as an immunogen.

(2) Immunization

To a 1 mg/ml suspension of the heat-denatured rhIL-2 in saline was added the same amount of Freund's complete adjuvant, and mice (females, n = 10) were started to be immunized with the resulting mixture (0.1 mg/0.2 ml/mouse) subcutaneously at their backs and abdomens. Supplemental immunization was carried out by inoculating the mice with a mixture of the immunogen and the same amount of Freund's incomplete adjuvant 5 times at 2 to 3-week intervals.

(3) Cell Fusion

The spleens were taken out of the mice 3 days after the final immunization, and a spleen cell suspension (containing about $10^8$ cells) was prepared by a method known in the art. Then, $2 \times 10^7$ mouse myeloma cells (P3U1) were added thereto, and cell fusion was carried out by using PEG 6,000 in accordance with the method of Köhler and Milstine [Nature 256, 495 (1975)]. After the cell fusion had been completed, the cell mixture solution was suspended in HAT medium containing hypoxanthine, aminopterin and thymidine, and cultivated for 10 days. Immediately after selection of parent cells was completed, the medium was exchanged for HT medium prepared by removing aminopterin from HAT medium, and cultivation was continued.

(4) Selection and Cloning of Hybridoma Cells

Using a microtiter plate on which the rhIL-2 was adsorbed by a solid phase, the antibody titer of the culture supernatant of hybridoma cells was measured by the ELISA. The appearance of the hybridoma cells was observed 10 to 20 days after fusion, and an antibody which specifically bound with the rhIL-2 was found. The hybridoma cells having particularly high antibody titer were cloned by the limiting dilution method.

The culture supernatant of the cloned hybridoma cells was similarly screened by the EIA to select hybridoma cells having high binding ability with the rhIL-2.

Using the hybridoma cells, immune complexes with various amounts of the rhIL-2 were obtained in a manner similar to those of Examples 3 and 4 mentioned below, and the resulting complexes were further given to the mice in a manner similar to that shown in Example 2. The distribution volume at this time was calculated, and hybridoma cells producing an antibody which reduced the distribution volume of the rhIL-2 were screened.

As a result, mouse hybridoma cell HRL1-52 (IFO 50222, FERM BP-2747) was obtained which specifically bound with the rhIL-2 and reduced the distribution volume of the rhIL-2 when the immune complex thereof was given to the mice. Its immunoglobulin class and subclass were examined by the Ouchterlony test. Both of them were found to be IgG$_1$.

Example 2 Preparation of Antibody

To each Balb/C mouse (female) was intraperitoneally given 0.5 ml of mineral oil. After one week, $10^6$ mouse hybridoma cells (HRL1-52) (IFO 50222, FERM BP-2747) obtained in Example 1 were intraperitoneally given thereto. After 10 days to 2 weeks from hybridoma cell administration, the ascites was collected. 100 ml of the resulting ascites was centrifuged to take out a supernatant. The supernatant was filtered through a 5-μm filter, and exchange for 10 mM MES buffer by gel filtration using PD-10 (Pharmacia) was carried out. Then, the resulting solution was subjected to liquid chromatography (AB$^R$ Column, J. T. Baker), thereby obtaining 114 mg of a desired antibody. The resulting antibody was applied to liquid chromatography (column: Asahipak GS-520, Asahi Chemical Industry; transfer phase: Dulbecco phosphate buffered saline; flow rate: 1 ml/min; detection: OD$_{280}$). As a result, a single peak was obtained as shown in Fig. 1. This retention time agreed with that of commercial mouse IgG (Chrompure Mouse IgG, whole molecule, Jackson Immunoresearch Lab. Inc.).

The binding ability of the monoclonal antibody with the rhIL-2 was confirmed by the ELISA generally known in the art. Namely, after the rhIL-2 was adsorbed by an immunoplate, the monoclonal antibody was added thereto. Then, HRP labeled anti-mouse IgG was added thereto, and the binding ability with the rhIL-2 was confirmed by the color development of o-phenylenediamine.

Example 3 Preparation of Immune Complex (Liquid)

To 1 ml of 1 mg/ml solution of the anti-IL-2 monoclanal antibody obtained in Example 2 was added 0.05 ml, 0.1 ml, 0.2 ml or 1 ml of a 1 mg/ml solution of the rhIL-2 to give a mole ratio of the anti-IL-2 monoclonal antibody to the rhIL-2 of about 2:1, 1:1, 1:2 or 1:10. Analysis of these mixtures by HPLC (column: Asahipak GS-520) revealed that an immune complex was formed by about 2 moles of the rhIL-2 to 1 mole of the anti-IL-2 monoclonal antibody and that the above mole ratio in the immune complex was about 1:2.

An immune complex was prepared by mixing 0.2 ml of the 1 mg/ml solution of the IL-2 with 1 ml of the 1 mg/ml solution of the anti-IL-2 monoclonal antibody. The application of the resulting immune complex to the above-mentioned HPLC (column: Asahipak GS-520) proved that no unbound rhIL-2 and no unbound monoclonal antibody existed (Fig. 2).

At this time, the immune complex had EIA activity equivalent to that of the rhIL-2.

The biological activity of the resulting immune complex was examined by the MTT method [H. Tada et al., Journal of Immunological Methods 93, 157-165 (1986)]. As a result, the specific activity of the immune complex per rhIL-2 was identical to that of the rhIL-2 itself. Namely, this revealed that the immune complex did not neutralize the biological activity of the rhIL-2.

Example 4 Preparation of Immune Complex (Solid)

With 0.2 ml of the 1 mg/ml solution of the rhIL-2 was mixed 1 ml of the 1 mg/ml solution of the anti-IL-2 monoclonal antibody, and water was evaporated by a Centriback concentrator to obtain a solid immune complex. At this time, the rotor portion of the Centriback concentrator was not heated. The cooling trap portion was adjusted to -90°C and the pressure is reduced to 5 mmHg or less by a vacuum pump. Then, 1 ml of distilled water was added thereto to redissolve it. At this time, the immune complex had EIA activity equivalent to that of the rhIL-2.

While not to be limited by this example, the solid immune complexes can be prepared in liquid mixing the rhIL-2 with the anti- IL-2 monoclonal antibodies in a mole ratio of about 2:1 and evaporating water with Speed-Vac Concentrator (SAVANT) or a lyophilizer.

Example 5 Preparation of F(ab')$_2$ (Liquid)

The anti-IL-2 monoclonal antibody and pepsin (Sigma) were mixed and reacted with each other at pH 3.5 at 37°C for 18 hours. After the reaction was stopped, the reaction product was subjected to a protein A column to obtain F(ab')$_2$ of the anti-IL-2 monoclonal antibody. The F(ab')$_2$ of the anti-IL-2 monoclonal antibody exhibited binding ability with the rhIL-2 by the ELISA shown in Example 2.

Example 6 Preparation of rhIL-2-F(ab')$_2$ Immune Complex (Liquid)

To 1 mole of the F(ab')$_2$ of the anti-IL-2 monoclonal antibody obtained in Example 5 was added 2 moles of the rhIL-2, thereby obtaining an immune complex of rhIL-2 anti-IL-2 monoclonal antibody F(ab')$_2$.

The resulting immune complex had activity as the rhIL-2, when examined by the EIA and the MTT method.

Example 7 Preparation of F(ab')$_2$ Immune Complex (Solid)

Water was evaporated by the Centriback concentrator from the immune complex of the anti-IL-2 monoclonal antibody F(ab')$_2$ and the rhIL-2 which had been obtained in liquid form, thereby obtaining a solid immune complex of the F(ab')$_2$ and the rhIL-2.

The resulting immune complex had activity as the rhIL-2, when examined by the EIA and the MTT method.

Example 8 Preparation of F(ab')$_2$ Immune Complex (Solid)

Water was evaporated by a lyophilizer from the immune complex of the anti-IL-2 monoclonal antibody F(ab')$_2$ and the rhIL-2 which had been obtained in liquid form, thereby obtaining a solid immune complex of the F(ab')$_2$ and the rhIL-2.

Example 9

Using monocyte colony-stimulating factor (M-CSF), an anti-CSF monoclonal antibody is obtained as with Examples 1 and 2, and a CSF-anti-CSF monoclonal antibody immune complex is obtained in the manner of Example 3.

Example 10

Using thyrotropin-releasing hormone (TRH), an anti-TRH monoclonal antibody is obtained as with Examples 1 and 2, and a TRH-anti-TRH monoclonal antibody immune complex is obtained in the manner of Example 3.

Example 11

Using oxenedorone, an anti-oxenedorone monoclonal antibody is obtained as with Examples 1 and 2, and an oxenedorone-anti-oxenedorone monoclonal antibody immune complex is obtained in the manner of Example 3.

Example 12

Using enkephalin, an anti-enkephalin monoclonal antibody was obtained as with Examples 1 and 2, and an enkephalin-anti-enkephalin monoclonal antibody immune complex was obtained in a manner similar to that of Example 3.

Example 13

Using erythropoietin, an anti-erythropoietin monoclonal antibody is obtained as with Examples 1 and 2, and an erythropoietin-anti-erythropoietin monoclonal antibody immune complex is obtained in the manner of Example 3.

Example 14

Using leuprorelin, an anti-leuprorelin monoclonal antibody is obtained as with Examples 1 and 2, and an leuprorelin-anti-leuprorelin monoclonal antibody immune complex is obtained in the manner of Example 3.

Experimental Example 1 Administration to Rats (iv)

The immune complex obtained in Example 3 was intravenously given to rats in a dose equivalent to 100 μg of the rhIL-2. The blood was withdrawn with time to measure the serum rhIL-2 concentration. The results are indicated by -●- in Fig. 3. As a control experiment, in lieu of the IL-2-anti-IL-2 monoclonal antibody, a mixture of the rhIL-2 and commercial mouse IgG was intravenously given to rats in a dose equivalent to 100 µg of the rhIL-2. The results are indicated by -o- in Fig. 3.

The changes with time in serum rhIL-2 concentration thus measured were pharmacokinetically analyzed. As a result, when the immune complex was given, the distribution volume of the rhIL-2 was about one-seventh (about 0.14 time) that in the control experiment. Namely, the serum rhIL-2 concentration was about 7 times higher than that in the control experiment. On the other hand, when this immune complex was given, the elimination rate constant of the rhIL-2 was not different from that in the control experiment. Namely, this shows that the half-life was unchanged as the immune complex.

Experimental Example 2 Administration to Mice (iv)

The immune complex of the rhIL-2 and the anti-IL-2 monoclonal antibody which was obtained in Example 4 was dissolved in water, and the resulting aqueous solution was intravenously given to mice in a dose equivalent to 10 µg of the rhIL-2. The blood was withdrawn with time to measure the plasma rhIL-2 concentration. As a control experiment, in lieu of the IL-2-anti-IL-2 monoclonal antibody complex, a mixture of the rhIL-2 and commercial mouse IgG was intravenously given to mice in a dose equivalent to 10 µg of the rhIL-2. The changes with time in serum rhIL-2 concentration thus measured were pharmacokinetically analyzed. The results are shown in Table 1.

### Table 1

#### Pharmacokinetic Parameters of the Immune Complex

#### of rhIL-2 and Anti-rhIL-2 Monoclonal Antibody

| Parameter | rhIL-2 | Immune complex |
|---|---|---|
| Distribution volume (ml/kg) | 600 | 70 |
| Elimination rate constant (l/h) | 2 | 2 |

1-Compartment model intravenous administration

As shown in Table 1, the results of analysis proved that the distribution volume of the rhIL-2 of the immune complex was about one-eighth (about 0.12 times) that in the control experiment. This shows that when the immune complex used in the experiment was given, the distribution of the rhIL-2 was changed, compared to the case that the aqueous solution of the rhIL-2 was given. The elimination rate constant of the rhIL-2 was not different from that in the control experiment. This shows that the half-life was not changed.

Experimental Example 3 Administration to Mice (sc)

The immune complex obtained in Example 4 was dissolved in water, and the resulting aqueous solution was subcutaneously given to mice in a dose equivalent to 10 µg of the rhIL-2. The blood was withdrawn with time to measure the blood rhIL-2 concentration. As a control experiment, in lieu of the complex, a mixture of the rhIL-2 and commercial mouse IgG was similarly given to mice. The results are shown in Fig. 4. Referring to Fig. 4, -o- and -●- indicate the results for the rhIL-2 and the immune complex, respectively. For the immune complex, the blood concentration was as high as 0.3 ng/ml or more for 24 hours or more, as shown in Fig. 4.

Experimental Example 4 Anti-Tumor Effect in Mice

Tumor cells (Meth-A) were implanted in mice (Bulb/C, 7 weeks old, females). An aqueous solution of the immune complex obtained in Example 4 was subcutaneously given thereto in a dose equivalent to 10 μg/mouse/day of the rhIL-2 once a day for 12 days from 7 days after implantation. As a control experiment, the rhIL-2 was subcutaneously given to mice in a dose of 10 μg/mouse in lieu of the IL-2-anti-IL-2 monoclonal antibody immune complex. No treatment was given to the control mice. Twenty-one days after implantation, the tumor cells were removed from the mice to measure their weight, and the ratio (T/C) of the weight of the tumor cells removed from the mice given the immune complex to the weight of the tumor cells removed from the control mice was determined. As a result, when the rhIL-2 was given, the T/C was 57%. In contrast, when the immune complex was given, the T/C was 16%. This shows that the immune complex highly enhances the effectiveness of the rhIL-2.

The above Experimental Examples 1 and 2 revealed that when the immune complexes of the present invention were given to animals, the distribution volume of the rhIL-2 was reduced, compared to the case that the rhIL-2 was given alone, and that the distribution of the rhIL-2 was changed. The above Experimental Examples 3 and 4 further revealed that the effectiveness of the rhIL-2 was highly enhanced by giving these immune complexes.

Example 15 Preparation of an Immune Complex (Solid) in which Anti-IL-2 Monoclonal Antibody is Incorporated in Excess

1 ml of a 10 mg/ml solution of the anti-IL-2 monoclonal antibody obtained in Example 2 was sufficiently mixed with 0.2 ml of a 1 mg/ml solution of the rhIL-2 at room temperature. Next, water was evaporated from the mixture by SpeedVac Concentrator at room temperature at a cold trap temperature of -80°C under 5 torrs or less to obtain a powdery preparation containing an immune complex having a monoclonal antibody:rhIL-2 ratio (mole ratio) of about 5:1 as an immune complex-containing injection. To this preparation was added 1 ml of distilled water to redissolve it. At this time, the immune complex had EIA activity equivalent to that of the rhIL-2.

Experimental Example 5 Administration to Mice (sc)

To the powdery immune complex-containing preparation obtained in Example 15 was added 1 ml of distilled water to redissolve it, followed by dilution with phosphate buffered saline (PBS). Then, the resulting solution was subcutaneously given to mice in a dose equivalent to 10 μg of the rhIL-2. The blood rhIL-2 concentration after 3 hours from administration was about 3.5 times that when the immune complex obtained in Example 4 was given to the mice (Experimental Example 3).

Example 16

Using granulocyte colony stimulating factor (G-CSF), an anti-G-CSF monoclonal antibody is obtained as with Examples 1 and 2, and an G-CSF-anti-G-CSF monoclonal antibody immune complex is obtained in the manner of Example 3.

Reference Example 1

Balb/c mice previously given intraperitoneally 0.5 ml of mineral oil were inoculated intraperitoneally with $5 \times 10^6$ cells of anti-urokinase MoAb producing hybridoma UK1-3, UK1-87 or UK1-6. After about 10 to 15 days, the pool of ascites was observed. The antibodies were purified by method known in the art. Namely, after fractionation with 40 to 50% saturated ammonium sulfate, the fraction was subjected to DEAE-cellulose chromatography and protein A column chromatography to obtain the corresponding anti-urokinase MoAb antibody UK1-3, UK1-87 or UK1-6. These antibodies were eluted by HPLC (column: Asahipak GS-520, eluent: PBS, flow rate: 1 ml/min) at 10.9 minutes, which agreed with the retention time of commercial mouse IgG.

Reference Example 2 Fibrinolysis Neutralization Test

A diluted solution of a test antibody was added to an urokinase solution having a final concentration of 25 ng/ml, followed by reaction at 37°C for 1 hour. Then, the reaction mixture solution was poured in an amount of 5 μl/well into each well of a fibrin agarose plate. After standing at 37°C for 2 to 6 hours, the dissolution unevenness (diameter) of fibrin was measured to determine the neutralization ability of an MoAb contained in the diluted solution of the test antibody to the enzyme activity of UK1.

Reference Example 3 Urokinase Enzyme Activity Neutralization Test

A test antibody solution was added to an urokinase solution having a final concentration of 1.78 µg/ml, followed by reaction at room temperature for 30 minutes. Then, synthetic peptide substrate S-2444 (1 mM, pyroglutamylglycylarginyl p-nitroanilide, Kavi Vitrum (Sweden) was added thereto. After reaction was further conducted at 37°C for 15 minutes, liberated p-nitroanilide was determined by the absorbance at 405 nm.

Reference Example 4 EIA of UK

The UK concentration was determined by the sandwich EIA using a goat anti-UK antibody as a primary antibody and HRP-linked rabbit anti-UK (Fab') as a secondary antibody.

Example 17 Preparation of Immune Complex (Liquid)

0.63 ml of a 10 mg/ml solution of anti-urokinase MoAb antibody UK1-3 described in European Patent Publication No. 363712, the disclosure of which is herein incorporated by reference, was mixed with 0.7 ml of a 1 mg/ml solution of high molecular weight urokinase (Nippon Seiyaku Seizo) in phosphate buffered saline so as to give a mole ratio of anti-urokinase MoAb antibody UK1-3 to urokinase of 3:1, thereby obtaining an immune complex. An assayed value for this immune complex by the sandwich method using anti-mouse IgG as a primary antibody and HRP-linked rabbit anti-UK (Fab') as a secondary antibody was not different from the assayed value obtained in Reference example 4. This fact proved that all urokinase existed as the immune complex, and further that the anti-urokinase immune complex could be determined by the EIA of UK described in Reference Example 4 as with urokinase alone. When the biological activity of the immune complexes obtained by the methods described in Reference Examples 2 and 3 was assayed, the specific activity of urokinase of the immune complexes was equal to that of urokinase itself. Namely, this revealed that the immune complexes did not neutralize the biological activity of urokinase.

Example 18 Preparation of Immune Complex (Solid)

Water was evaporated by lyophilization from the immune complex obtained in Example 17 to obtain a solid immune complex. To this immune complex was added 1 ml of distilled water to redissolve it. At this time, when the EIA activity was determined in a manner similar to that of Reference Example 4, the immune complex had activity equivalent to that of urokinase.

Example 19 Preparation of F(ab')2 (Liquid)

Anti-urokinase MoAb antibody UK1-3 and pepsin (Sigma) were mixed and reacted with each other at pH 3.5 at 37°C for 18 hours. After the reaction was stopped, the reaction product was subjected to a protein A column to obtain $F(ab')_2$ of anti-urokinase MoAb antibody UK1-3. This $F(ab')_2$ had binding ability with urokinase. The anti-urokinase MoAb UK1-3 F(ab') was mixed with high molecular weight urokinase (Nippon Seiyaku Seizo) in phosphate buffered saline so as to give a mole ratio of 3:1, thereby obtaining an immune complex. At this time, when the EIA activity was determined in a manner similar to that of Reference Example 4, the immune complex had activity equivalent to that of urokinase.

Example 20 Preparation of F(ab') Immune Complex (Solid)

Water was evaporated by lyophilization from the immune complex obtained in Example 19 to obtain a solid immune complex. To this immune complex was added 1 ml of distilled water to redissolve it.

Example 21 Preparation of Immune Complex (Liquid)

0.63 ml of a 10 mg/ml solution of anti-urokinase MoAb antibody UK1-87 described in European Patent Publication No. 363712 was mixed with 0.7 ml of a 1 mg/ml solution of high molecular weight urokinase (Nippon Seiyaku Seizo) in phosphate buffered saline so as to give a mole ratio of anti-urokinase MoAb antibody UK1-87 to urokinase of 3:1, thereby obtaining an immune complex.

Example 22 Preparation of Immune Complex (Liquid)

0.63 ml of a 10 mg/ml solution of anti-urokinase MoAb antibody UK1-6 described in European Patent Publication No. 363712 was mixed with 0.7 ml of a 1 mg/ml solution of high molecular weight urokinase (Nippon Seiyaku Seizo) in phosphate buffered saline so as to give a mole ratio of anti-urokinase MoAb antibody UK1-6 to urokinase of 3:1, thereby obtaining an immune complex.

Example 23 Preparation of Immune Complex (Liquid)

Anti-urokinase MoAb antibody UK1-3 described in European Patent Publication No. 363712 was mixed with double stranded low molecular weight urokinase (JCR) in phosphate buffered saline so as to give a mole ratio of 3:1, thereby obtaining an immune complex.

Example 24 Preparation of Immune Complex (Liquid)

Anti-urokinase MoAb antibody UK1-3 described in European Patent Publication No. 363712 was mixed with prourokinase in phosphate buffered saline so as to give a mole ratio of 3:1, thereby obtaining an immune complex.

Experimental Example 6 Administration to Rats (iv)

The immune complex obtained in Example 17 was intravenously given to rats (n = 5) in a dose equivalent to 100 µg of urokinase. The blood was withdrawn with time to measure the plasma urokinase concentration. As a control experiment, urokinase alone was intravenously given to rats in a dose of 100 µg. The results are shown in Fig. 5. Referring to Fig. 5, -○- and -o- indicate the results for urokinase alone and the immune complex, respectively.

The changes with time in plasma urokinase concentration thus measured were pharmacokinetically analyzed. As a result, the half-life (72 minutes) of urokinase of the immune complex was increased to about 2.6 times that (28 minutes) in the control experiment, and the distribution volume (27 ml) was reduced to about 0.3 times that (91 ml) in the control experiment.

Experimental Example 7 Administration to Rats (sc)

The immune complex obtained in Example 17 was subcutaneously given to rats (n = 5) in a dose equivalent to 100 µg of urokinase. The blood was withdrawn with time to measure the plasma urokinase concentration. As a control experiment, the urokinase was similarly given to mice. The results are shown in Fig. 6. Referring to Fig. 6, -o- and -□- indicate the results for the immune complex and urokinase alone, respectively. For the immune complex, the plasma concentration was as high as 0.1 µg/ml or more for 48 hours or more, as shown in Fig. 6. For the urokinase alone, however, the plasma concentration after 8 hours from administration was markedly lower than 0.1 µg/ml.

The following references are referred to for their disclosures at various points in the application.

The Pharmacological Basis of Therapeutics, (1985), MacMillan Publishing Company, New York, NY pp. 22-28

Journal of Immunology 135, 2865-2875 (1985)
British Journal of Cancer 47, 123-133 (1983)
Cancer Immunology and Immunotherapy 30, 71-80 (1989)
Japanese Patent Unexamined Publication No. 60-226821/1985 which corresponds to EP-154,316
PCT/US86/01252 (WO8700/056A)
Japanese Patent Unexamined Publication No. 60-502104;
PCT/US84/01389 (WO85/00974)
Cancer Research 45, 2421-2424 (1985)
Japanese Patent Unexamined Publication No. 61-78799/1986 which corresponds to EP-176,299
Japanese Patent Unexamined Publication No. 60-126088/1985
Japanese Patent Unexamined Publication No. 59-93093/1984 which corresponds to U.S. Patent No. 4,518,584
Japanese Patent Unexamined Publication No. 60-115528/1985 which corresponds to EP-145,390
Primer of Pharmacokinetics by Microcomputers, p.159, Nankodo (1983)
Proc. Natl. Acad. Sci. USA 85, 4852 (1988)

Journal of Immunological Methods 93, 157-165 (1986)

**Claims**

1.  An immune complex comprising a biologically active substance and a monoclonal antibody to the biologically active substance, the monoclonal antibody being able to reduce the distribution volume of said biologically active substance 0.5 times or less compared to the case in which said substance is used alone.

2.  A pharmaceutical composition for administration to a host which comprises an effective amount of the immune complex of claim 1.

3.  A pharmaceutical composition in accordance with claim 2, which is a powdery dried product.

4.  An immune complex in accordance with claim 1, in which said biologically active substance is a cytokine.

5.  An immune complex in accordance with claim 4, in which said cytokine is interleukin-2.

6.  An immune complex in accordance with claim 1, in which said biologically active substance is an enzyme.

7.  An immune complex in accordance with claim 6, in which said enzyme is urokinase.

8.  An immune complex in accordance with claim 1, in which said monoclonal antibody belongs to immunoglobulin G.

9.  An immune complex in accordance with claim 1, in which said monoclonal antibody is a fragment having antibody activity of immunoglobulin G.


**Patentansprüche**

1.  Immun-Komplex, umfassend eine biologisch aktive Substanz und einen monoklonalen Antikörper gegen die biologisch aktive Substanz, wobei der monoklonale Antikörper fähig ist, das Verteilungs-Volumen der biologisch aktiven Substanz auf das 0,5-fache oder weniger zu reduzieren, verglichen mit dem Fall, in dem die Substanz allein verwendet wird.

2.  Pharmazeutische Zusammensetzung zur Verabreichung an einen Wirt, umfassend eine wirksame Menge des Immun-Komplexes des Anspruchs 1.

3.  Pharmazeutische Zusammensetzung nach Anspruch 2, die ein pulvriges getrocknetes Produkt ist.

4.  Immun-Komplex nach Anspruch 1, worin die biologisch aktive Substanz ein Cytokin ist.

5.  Immun-Komplex nach Anspruch 4, worin das Cytokin Inter-leukin-2 ist.

6.  Immun-Komplex nach Anspruch 1, worin die biologisch aktive Substanz ein Enzym ist.

7.  Immun-Komplex nach Anspruch 6, worin das Enzym Urokinase ist.

8.  Immun-Komplex nach Anspruch 1, worin der monoklonale Antikörper zu Immunoglobulin G gehört.

9.  Immun-Komplex nach Anspruch 1, worin der monoklonale Antikörper ein Fragment mit Antikörperwirkung des Immunoglobulins G ist.


**Revendications**

1.  Complexe immunitaire comprenant une substance biologiquement active et un anticorps monoclonal envers la substance biologiquement active, l'anticorps monoclonal étant capable de réduire à la moitié ou moins le volume de distribution de ladite substance biologiquement active par rapport au cas où ladite substance est utilisée seule.

2. Composition pharmaceutique pour administration à un hôte, qui comprend une quantité effective du complexe immunitaire de la revendication 1.

3. Composition pharmaceutique selon la revendication 2, qui est sous forme d'un produit séché pulvérulent.

4. Complexe immunitaire selon la revendication 1, dans lequel ladite substance biologiquement active est une cytokine.

5. Complexe immunitaire selon la revendication 4, dans lequel ladite cytokine est l'interleukine-2.

6. Complexe immunitaire selon la revendication 1, dans lequel ladite substance biologiquement active est une enzyme.

7. Complexe immunitaire selon la revendication 6, dans lequel ladite enzyme est l'urokinase.

8. Complexe immunitaire selon la revendication 1, dans lequel ledit anticorps monoclonal appartient à l'immunoglobuline G.

9. Complexe immunitaire selon la revendication 1, dans lequel ledit anticorps monoclonal est un fragment comportant l'activité d'anticorps de l'immunoglobuline G.

# Fig. 1

I — 1

OD 280 nm

11.547

10.483

0

10

Time (minute)

I — 2

OD 280 nm

11.551

0

10

Time (minute)

19

## Fig. 2

Fig. 3

Fig. 4

Fig. 5

EP 0 514 544 B1

Fig. 6